(19) Europäisches Patentamt
European Patent Office
Office européen des brevets

(11) Publication number: 0 062 322
A1

(12) # EUROPEAN PATENT APPLICATION

(21) Application number: 82102819.8

(22) Date of filing: 02.04.82

(51) Int. Cl.³: C 07 C 102/00, C 07 C 103/50, C 07 D 211/58, C 07 D 295/18

(30) Priority: 03.04.81 US 250826

(43) Date of publication of application: 13.10.82 Bulletin 82/41

(84) Designated Contracting States: BE CH DE FR GB IT LI NL

(71) Applicant: The B.F. GOODRICH Company, Dept. 0015 WHB-6 500 South Main Street, Akron, Ohio 44318 (US)

(72) Inventor: Lai, John Ta-Yuan, 663 Tollis Parkway, Broadview Heights Ohio 44147 (US)

(74) Representative: von Kreisler, Alek, Dipl.-Chem. et al, Deichmannhaus am Hauptbahnhof, D-5000 Köln 1 (DE)

(54) **Improved process for synthesis of hindered amine stabilizers for polymeric materials.**

(57) Process for the synthesis of hindered polysubstituted α-amino acetamides by reaction of a secondary amine with an α-halo acetamide in a basic organic reaction medium and in the presence of a phase transfer catalyst. The product thus obtained can comprise a multifunctional compound having one or more hindered amino moieties. These compounds are highly effective as UV stabilizers in a variety of plastics, especially the alpha monoolefins.

EP 0 062 322 A1

# IMPROVED PROCESS FOR SYNTHESIS OF HINDERED AMINE STABILIZERS FOR POLYMERIC MATERIALS

## BACKGROUND OF THE INVENTION

Field of the Invention - This invention relates to a process. More specifically, this invention is directed to the synthesis of hindered amines by phase transfer catalyzed condensation of a primary or secondary amine with an α-halo-acetamide.

Description of the Prior Art - The increasing use of polymers in the place of the more traditional types of structural materials, (e. g. wood, metals, etc.) has necessitated the compounding of such polymers with a variety of stabilizers in order to enhance their ability to withstand prolonged exposure to a variety of degradative forces. Degradation of such environmentally sensitive polymers can be caused by exposure to light, heat and/or air. Such degradation is usually manifest by either a partial or total loss of structural integrity, changes in light transmission properties, changes in color, loss or reduction in flexibility and/or resiliency, or any combination of the above phenomenon. As will be appreciated, the stabilizers which are used in conjunction with the above polymeric materials, in addition to providing protection against such degradative forces, must also be compatible with the aesthetic properties of the polymeric article formed from such materials and be effective at low concentrations. The economics of the marketplace dictate that these stabilizers be relatively inexpensive and capable of preparation from readily available starting materials by simple and straightforward synthesis techniques.

The prior art is replete with both patents and technical articles describing various stabilizers suitable for use in structural/engineering plastics and in various synthetic fibers. The hindered amine stabilizers are prominently mentioned as suitable in

the stabilization of such materials against ultraviolet light degradation. Illustrative of these hindered amines are the decahydroquinolines disclosed in U. S. Patents 3,919,234; 3,920,659; 3,928,330; 4,069,195; and 4,073,770; the 1,5-diazacycloalkan-2-ones disclosed in U. S. Patent 4,207,228; and, the 1,4-diazacycloalkan-2-ones disclosed in U. S. Patents 4,167,512 and 4,240,961. These hindered amine stabilizers can be prepared in various ways and from various materials.

The preparation of the U.V. stabilizers disclosed in U. S. Patents 4,240,961 and 4,190,571 is reportedly achieved by phase transfer catalyzed reaction of certain appropriate starting materials. Phase transfer catalysis initiated synthesis is also reportedly effective in the synthesis of other types of stabilizer; namely, the synthesis of antioxidants and compounds which enhance a polymer's resistance to thermal degradation.

The first disclosure relating to the phase transfer catalyzed synthesis of this latter class of stabilizers is by Dr. J. T. Lai in a paper presented before the A.C.S. National Meeting on March 19, 1978 in Houston, Texas entitled, "Synthesis of α-Aminoamides from Phase Transfer Catalyzed Reactions". The subject matter of Dr. Lai's paper is also described in copending patent application 916,639 filed June 19, 1978. The title compounds of Dr. Lai's paper are prepared by reaction of aniline, and its para-substituted derivatives, with chloroform, acetone and sodium hydroxide in the presence of a phase transfer catalyst.

Dr. Lai's synthesis proceeds by reaction of the chloroform with base thereby generating a trichloromethide ion which can subsequently combine with the ketone (or aldehyde) forming an oxirane intermediate. This intermediate combines with the aniline, or its para-substituted derivatives, and optionally, with another primary or secondary amine yielding the desired reaction product.

Where the foregoing synthesis is carried out with aniline, or para-substituted aniline, only one product will result and such product will be symmetrical. In the event that a primary or secondary amine is also present, in addition to aniline, or a para-substituted aniline, the reaction can still be directive for a single product, depending upon the concentration of the primary or secondary amine, (a stoichiometric excess being required), relative to the aniline, or para-substituted aniline. The product obtained in this latter situation will be asymmetrical in the sense that one end of the resultant compound will be substituted by the aniline and the other end by the primary or secondary amine. In both of the above synthesis, that is the synthesis of symmertrical or asymmetrical compounds in which aniline, or parasubstituted aniline, is a reactant, only one reaction product will result. In the event the foregoing synthesis is repeated without aniline, or a para-substituted aniline, being present, the primary or secondary amine in the charge will react with the oxirane intermediate randomly (i.e., random attachment at both of the potentially reactive sites) yielding a mixture containing four products.

It is, thus, readily apparent, that if one desires to use phase transfer catalyzed synthesis of compounds such as described by Lai, aniline, or para-substituted aniline, would appear to be a required reactant to avoid the formation of product mixtures, (which are both difficult to separate and may contain compounds deficient in the desirable stabilizer activity).

But for the limitations imposed by the choice of aniline, or para-substituted aniline, as a reactant, the above synthesis would provide both an efficient and desirable process for preparation of useful stabilizer compounds.

## SUMMARY OF THE INVENTION

Accordingly, it is the object of this invention to remedy the above as well as related deficiencies in the prior art.

More specifically, it is the principal object of this invention to provide a process which is directive for the synthesis of α-substituted acetamides by phase transfer catalyzed reaction of a primary or secondary amine and an α-halo-acetamide.

It is yet another object of this invention to provide a process which is directive for the synthesis of symmetrically substituted hindered amines by phase transfer catalyzed reaction of a primary or secondary amine and an α-halo-acetamide.

The above and related objects are achieved by providing a process for the synthesis of polysubstituted α-amino acetamides of the formula

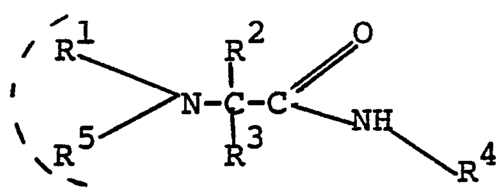

wherein $R^1$ and $R^4$ are independently selected from the group consisting of alkyl of 1-8 carbon atoms, aryl, cycloalkyl, piperidinyl, hindered piperidinyl and alkalene of 2-6 carbon atoms;

$R^2$ and $R^3$ are independently selected from the group consisting of hydrogen and alkyl of 1-6 carbon atoms; and

$R^5$ is hydrogen or alkyl of 1-6 carbon atoms and can, in conjunction with the acyclic substituents of $R^1$, form a heterocyclic group pendant from the α-carbon

by reaction, in the presence of a suitable phase transfer catalyst and alkali, of a primary or secondary amine with

an α-halo-acetamide. The terms "asymmetrical" and "symmetrical" as employed herein in the characterization of the substituted acetamide refers to similarity or dissimilarity in the substituents pendent from and/or which include the nitrogen atoms at the opposite ends of such compounds. These compounds are useful in the stabilization of photosensitive polymers against the degradative action of ultraviolet light.

## DESCRIPTION OF THE INVENTION INCLUDING PREFERRED EMBODIMENTS

The α-substituted acetamide described hereinabove can be prepared in accordance with the process of this invention by phase transfer catalyzed reaction, under suitable conditions and in suitable reaction medium, of a primary or secondary amine with an α-halo-acetamide.

The primary and secondary amines suitable for use in this process can be represented by the following formula:

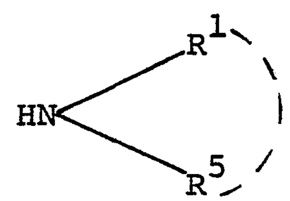

wherein $R^1$ is selected from the group consisting of alkyl of 1-8 carbon atoms, aryl, cycloalkyl, piperidinyl, partially or totally hindered piperidinyl, and alkalene of 2-6 carbon atoms, and $R^5$ is hydrogen, alkyl of 1-6 carbon atoms and can, in conjunction with the acyclic substituents of $R^1$, form a heterocyclic compound.

Representative of the primary and secondary amines which are within the scope of the foregoing formula and suitable for use in the process of this invention include: <u>1° amines</u>, t-butylamine, t-octylamine, aniline, alkyl substituted aniline (i.e. toluidine), isopropyl amine, propyl amine, 4-amino-2,2,6,6-tetramethyl-piperidine,

allyl amine; and, 2° amines, morpholine, N-methyl-aniline, diethyl amine.

The α-halo-acetamides suitable for use in the process of this invention can be represented by the following formula:

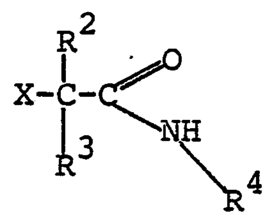

wherein $R^2$ and $R^3$ are independently selected from the group consisting of hydrogen and alkyl of 1-6 carbon atoms; $R^4$ is selected from the group consisting of alkyl of 1-8 carbon atoms, aryl, cycloalkyl, piperidinyl, partially or totally hindered piperidinyl, and alkalene of 2-6 carbon atoms; and X is chloride or bromide.

Representative of the α-halo-acetamides which are within the scope of the foregoing formula and suitable for use in the process of this invention include: α-chloro-α,α-dimethyl-N-phenylacetamide, α-bromo-α,α-dimethy-N-phenylacetamide, α-bromo-α,α-dimethyl-acetamide, N,N'[1,2-ethane-bis (α-bromo-α,α-diethyl acetamide)], and α,bromo-α,α-dimethyl-N-(2,2,6,6-tetramethyl-4-piperidinyl) acetamide.

The overall synthesis involved in the process of this invention involves initially charging a reactor, under the conditions hereinafter set forth with an appropriate amine, an α-halo-acetamide, base (i.e., an alkali metal hydroxide) and phase transfer catalyst, and thereafter heating the contents of the reactor for a predetermined interval. The synthesis proceeds generally as follows:

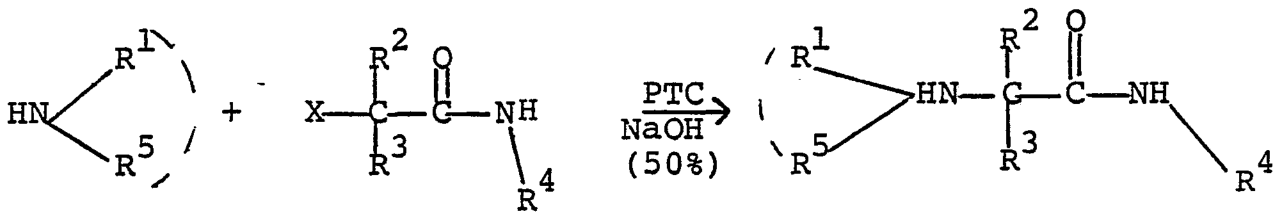


(1) (2) (3)

Although the foregoing reaction appears straight forward, the various reactants initially combine with one another to form certain intermediate compounds, which in turn react with one another to form the desired product. It is in the formation of these intermediate compounds which distinguishes this process from that previously disclosed by Dr. Lai in his A.C.S. presentation and in his copending application, Serial Number 916,639.

Initially, the α-halo-acetamide, referred to hereinabove, reacts with the sodium hydroxide in the charge to produce an imino-α-lactone as follows:

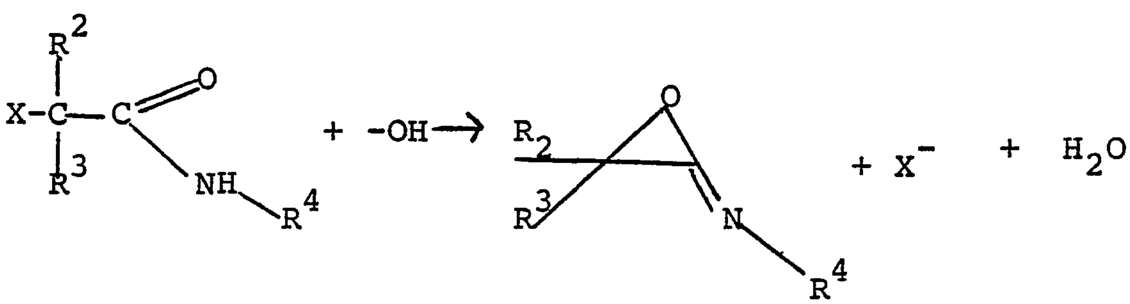

(2) (4)

This intermediate species differs significantly from the oxirane intermediate of the process previously disclosed by Lai in that the above imino-α-lactone has only one potentially reactive site for addition of the primary or secondary amine. Therefore, this imino-α-lactone and the primary and secondary amines, set forth hereinabove, can only react with one another as follows:

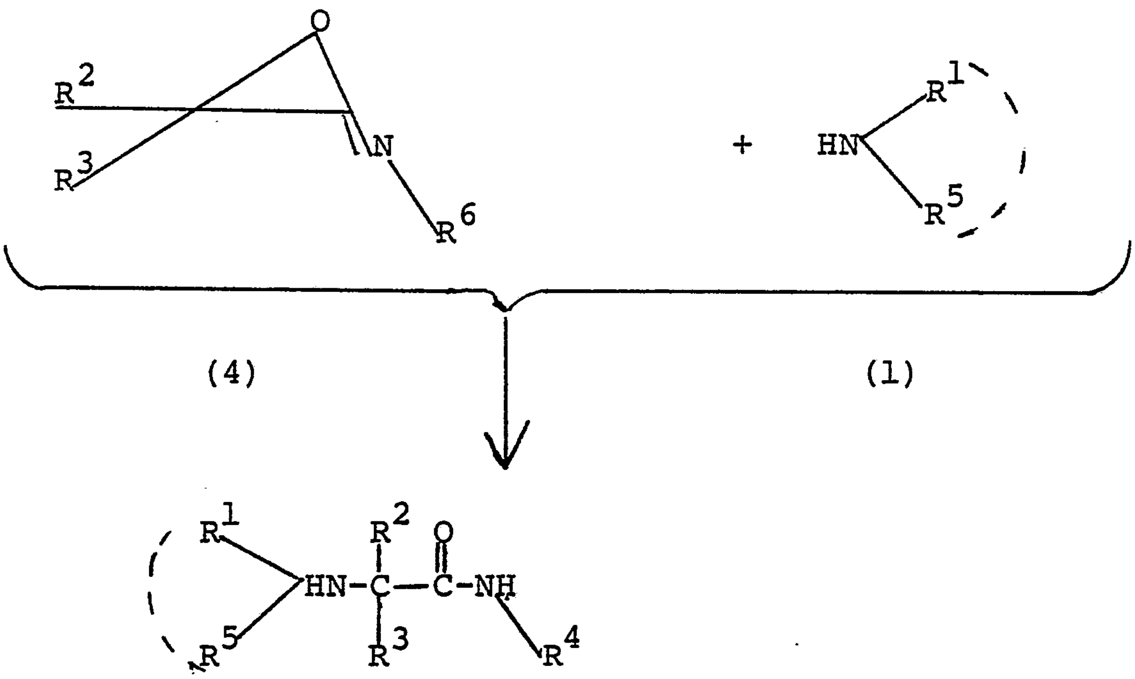

(4) (1)

(3)

As is apparent, the above series of reactions permits the formation of both symmetrical and asymmetrical compounds; does not lead to the formation of product mixture since the imino-α-lactone is only reactive toward the primary and secondary amine at a single site, the tertiary carbon; the reaction requires less base for the neutralization of the mineral acid formed incidental to the imino-α-lactone formation; and the reaction is decidedly less exothermic thereby permitting greater freedom of process control. By way of comparison, it will be appreciated that since the reaction of the oxirane intermediate with aniline, and substituted aniline, proceeds spontaneously in the presence of base and phase transfer catalyst and, the reaction temperature must be carefully controlled; the reaction temperature being preferably maintained between 0 to 10° C. In contrast to the above, the imino-α-lacetone intermediate, produced during the process of this invention, reacts only sluggishly with both the primary and secondary amine under ambient laboratory conditions and requires

heating of the reactor charge, preferably to a temperature in the range of from about 40 to 100° C, to drive the reaction, at an acceptable rate, in the direction of the desired product.

In the process of this invention, the relative concentration of the reactants to one another, and process conditions of this synthesis should be adequate to insure both a satisfactory rate of reaction and acceptable product yield. In general, the relative molar concentration of primary or secondary amine to α-halo-acetamide can range from about 1:1 to about 100:1. In the preferred embodiments of this invention, a stoichiometric excess of primary or secondary amine is required to drive the reaction in the direction of the desired product. The concentration of NaOH (present as a 50% aqueous solution, or in solid form) is generally not critical so long as sufficient base is present to transform the α-halo-acetamide to the corresponding imino-α-lactone and neutralize the mineral acid generated during the intermediate phase of the reaction. Good results have been obtained where the molar concentration of NaOH, (based upon a 50% aqueous solution), relative to α-halo-acetamide in the original charge, is in the range of from about 1:5. This relationship, of course, is somewhat misleading since the NaOH solution is preferably added incrementally to the reactor after all the reactants, including phase transfer catalyst, have been charged to the reactor.

The phase transfer catalysts which are suitable for use in the process of this invention, include those materials that can generally be described as onion salts of sulfur, or of any element of Group VA of the Periodic Table and which satisfy the following formula

$$R_nY^+X^-$$

wherein Y is selected from the group consisting of nitrogen, phosphorous and sulfur;
R can be one or more monovalent organic radicals bonded to Y by covalent linkage;
X is a counterion; and
n is an integer from 3 to 4
with the proviso, that when Y is pentavalent, that is nitrogen and phosphorous, then n=4, and when Y is tetravalent, that is sulfur, then n=3.

In an analogous manner, onium salts having certain multivalent organic substituents may be useful in this invention. Examples include multivalent organic radicals that include Y in a ring, and those that are bonded to more than one Y.

More preferred onium salts for use in this invention have the formula $(R_aR_bR_cR_dY^+)X^-$ wherein Y is N or P, and $R_a$-$R_d$ are monovalent hydrocarbon radicals preferably selected from the group consisting of alkyl, alkenyl, aryl, alkaryl, aralkyl, and cycloalkyl moieties or radicals, optionally substituted with suitable heteroatom-containing functional groups. The total number of carbon atoms in $R_a$, $R_b$, $R_c$, and $R_d$, if the salt is quaternary, should be at least 10 and is preferably in the range from about 15 to 40. No theoretical maximum number of carbon atoms for inclusion in the onium salts exists, although in general, about 70 carbon atoms represents the upper limit imposed by practical limitations. Since the liquid phases involved are both aqueous and organic, the number of the carbon atoms and structure of the onium salts are usually selected to impart to the salt a marked solubility in the organic phase. The onium salt itself is nonreactive with respect to all materials in the reaction mixture except the reactants themselves.

Most preferred onium salts are those in which Y is nitrogen and hydrocarbon radicals where $R_a$ is $C_2H_5$, and $R_b$,$R_c$ and $R_d$ are each selected from the group consisting of

n-$C_4H_5$; n-$C_5H_{11}$; mixed $C_5H_{11}$; n-$C_6H_{13}$; mixed $C_6H_{13}$; $C_6H_5$; $C_6H_5CH_2$; n-$C_8H_{17}$; n-$C_{12}H_{25}$; n-$C_{18}H_{37}$; mixed $C_8$-$C_{10}$ alkyl; and the like. However, $R^1$ may also be selected from $C_2H_5$, n-$C_3H_7$ and n-$C_4H_9$.

Various counterions may be used, including $Cl^-$, $Br^-$, $I^-$, $F^-$, $SO_4^{=}$, $HSO_4^-$ and the like. Most preferred is $Cl^-$.

The concentration of the onium salts, of the foregoing structure, which are present in the charge need only be sufficient to effectively catalyze the reaction of the amine and the α-halo-acetamide. In practice, the quantity of catalyst employed is in the range from about 0.01 mol to about 10 mols, and more preferably, for the usual practice of this invention, from about 1 to about 20 mpha (mols per 100 mols of primary or secondary amine) used. The amount of salt used is not critical, the optimum amount in each case being easily determined by simple trial and error. An amount greater than about 20 mpha is uneconomical and serves no useful purpose.

Typically, the foregoing reactants and catalyst are added to a reactor, such as a round-bottomed flask equipped with a reflux condenser, the reactor purged of air with an inert gas, and aqueous NaOH added incrementally while the exotherm of the reaction is initially controlled by immersion of the base of the flask in an ice bath. The reactor is also preferably equipped with a magnetic stirrer. The conditions prevailing during such process can be readily controlled and generally the reaction proceeds at atmospheric pressure. The reaction can proceed satisfactorily at temperatures in the range of from about 20°C to about 100°C, and most preferably from 40°C to 100°C.

Often times one or more of the reactants themselves (i.e., the primary or secondary amine) can serve as the medium for the conduct of the synthesis of this invention. Alternatively, such synthesis can also be

satisfactorily conducted in an organic solvent; provided, such solvent is inert toward both the reactants and product of the synthesis under the anticipated reaction conditions. Typical organic solvents which can be used in this process include the common aromatic and paraffinic solvents such as benzene, p-xylene, toluene, dichloromethane, chlorobenzene, cyclohexane and the like.

Once the desired compound has been prepared from the aforementioned materials in accordance with the above process, it can be readily recovered from the reaction medium by conventional means.

The compounds prepared as described above are highly effective in the stabilization of photodegradable polymeric material from the deteriorating effects of ultraviolet light.

The term "photodegradation" as used herein is intended as descriptive of any photo-induced changes in the physical, chemical and/or electrical properties of such organic polymeric materials upon their exposure to sufficient quanities of ultra-violet light. Such degradation can typically include cross-linking of the polymer, dehydrohalogenation, reduction in chain length, photooxidation and the like. Polymers which are especially sensitive to ultraviolet light degradation are materials which contain unsaturation along their respective backbones, such as cis-polyisoprene, styrene/butadiene copolymer, vinyl halide polymers, polyolefins, polyacetaldehydes, polyurethanes, ABS resins, polystyrene, polyacrylonitrile, polycarbonates, polyacrylates, poly-α-alkyl-acrylates, varnish, phenol-formaldehyde resins, polyepoxides, polyesters, and their respective blends and copolymers. The compounds prepared according to the process of this invention are especially effective in the stabilization of the poly-α-monoolefins such as polymers derived from ethylene, propylene, isobutylene, 1-butene, 1-pentene, 1-hexene, 4-methyl-1-pentene and the like.

In addition to the stabilizers prepared according to the process of this invention, a typical structural/engineering plastic can contain common compounding ingredients and additional stabilizers for the protection of such plastic against various other degradative forces and agents.

Representative compounding ingredients can include metal oxides, such as zinc, calcium and magnesium oxide, fatty acids such as stearic, lauric acid and the metal salts thereof; fillers such as calcium and magnesium carbonate, calcium and barium sulfonates, aluminum silicates, asbestos, and the like; plasticizers and extenders, such as dialkyl and diaryl organic acids, such as diisobutyl, diisooctyl, diisodecyl and dibenzyl oleates, stearates, sebacates, azelates, phthalates, and the like; ASTM Type 2 petroleum oils, paraffinic oils, castor oil, tall oil, glycerine, and the like; antioxidants, such as 2,6-di-t-butyl phenol), 2,2'-thio-bis-(4-methyl-6-t-butyl phenol), 2,2'-methylene-bis-6-t-butyl-4-ethyl phenol, 4,4'-butyldiene-bis-6-t-butyl-m-cresol, 2-(4-hydroxy-3,5-di-t-butylanilino-4,6-bis(octylthio)1,3,5-triazine, hexahydro-1,3,5-tris-β-(3,5-di-t-butyl-4-hydroxybenzyl)-isocyanurate, tetrakismethylene-3(3',5'-di-t-butyl-4'-hydroxyphenyl) propionate methane, distearylthiodipropionate, dilaurylthiodipropionate, tri(nonylphenyl) phosphite, tin thioglycolate, and the like; and other ingredients such as pigments, tackifiers, flame retardants, fungicides, and the like.

Addtional stabilizers which are especially preferred for use in combination with UV stabilizer, prepared as described herein, are the antioxidants. The inclusion within the polymer composition of an antioxidant, in addition to the UV stabilizer, confers upon the polymer composition stability against two of the more environmentally hostile degradative forces. The antioxidant can be present within the polymer composition

within the range of from about 0.1 to about 10 parts by weight per 100 parts by weight polymer, and preferably from about 0.2 to about 5 parts by weight per 100 parts by weight of polymer. Generally, the phenolic antioxidants are preferred for use in conjunction with the UV stabilizer of this invention.

Examples of phenolic antioxidants are 2,6-di-t-butyl-phenol; 2-methyl-4,6-dinonyl phenol; 2,6-di-t-butyl-p-cresol; 2,2'-methylenebis (4-methyl-6-t-butyl phenol); 1,1'-methylenebis (2-naphthol); 4,4'-methylenebis (2,6-di-t-butyl phenol); 4,4'-thiobis(6-t-butyl-m-cresol); and the like. Some of the more common and popular of the phenolic anti-oxidants are the esters having alkylhydroxyphenyl substituents; trazines having alkylhydroxyphenyl substituents; and isocyanurates having alkylhydroxyphenyl substituents.

Examples of phenolic antioxidants having alkylhydroxyphenyl substituents on an ester nucleus are disclosed in U.S. Pat. Nos. 3,330,859 and 3,627,725 and exemplified by di-lauryl α,α'-bis(3,5-di-t-butyl-4-hydroxybenzyl)malonate; compounds exemplified by tetrakis(methylene-3-(3',5'-di-t-butyl-4'-hydroxyphenyl) propionate)methane; and the like.

Examples of phenolic antioxidant compounds having alkyhydroxyphenyl substituents on a heterocyclic nucleus are triazine compounds such as disclosed in U.K. Pat. No. 977,589 and exemplified by 2,4,6-tris(4-hydroxy-3,5-di-t-butyl benzylthio)-1,3,5-triazine; disclosed in U.S. Pat. No. 3,706,740 and exemplified by 2,4,6-tris(3',5'-di-t-butyl-4'-hydroxybenzyl)-1,3,5-triazine; disclosed in U.S. Pat. No. 3,567,724 and exemplified by hexahydro-1,3,5-tris-β-(3,5-di-t-butyl-4-hydroxyphenyl)propionyl-s-triazine; disclosed in U.S. Pat. No. 3,694,440 and exemplified by 1,3,5-tris(4'-hydroxy-3',5'-di-t-butylphenylpropionyloxyethylthiopropionyl)hexahydro-1,3,5-triazine; and the like.

Examples of phenolic antioxidant compounds having alkylhydroxyphenyl substituents on an isocyanurate nucleus

are disclosed in U.S. Pat. No. 3,531,483 and exemplified by tris-(3,5-di-t-butyl-4-hydroxybenzyl)isocyanurate; disclosed in U.S. Pat. No. 3,678,047 and exemplified by 2,2',2"-tris(3,5-di-t-butyl-4-hydroxyphenyl)propionyloxy) ethyl isocyanurate; and the like.

Still other hindered phenols useful as thermal antioxidants are disclosed in U.S. Pat. No. 3,920,659, and in copending U.S. Patent applications Ser. No. 697,345 and Ser. No. 697,387 which are incorporated herein by reference as if fully set forth.

Ordinarily, a structural/engineering grade resin can be compounded with the various types of stabilizer materials described hereinabove in accord with standard mixing techniques and equipment; such as in a Banbury mixer, a Henschel mixer, a rubber mill, an extruder mixer or equivalent device. The various components of the composition may be physically intimately blended either in the absence of or in the presence of a common solvent; or in a solvent which is capable of dissolving the polymer component of the composition yet substantially incapable of dissolving the stabilizer ingredients. Typical of such solvent/dispersing agents include hexane or benzene. Subsequent to intimately dispersing the various components of the composition within one another, the dispersing agent (if any) can be removed by selective evaporation and the resultant resin recovered. The resin may thereafter be formed into useable products by a variety of standard techniques.

The ultra-violet light stability of the compositions of this invention is evaluated by exposing samples of a photosensitive plastic, with and without stabilizer, to a Xenon or carbon arc light in a Weather-Ometer operating at temperatures of about 60°C. The sample is considered to have been photodegraded when it has lost in excess of fifty percent (50%) of its tensile strength as determined by ASTM D 638-76. In a typical test

protocol, a pre-selected quantity of UV stabilizer, antioxidant and other optional processing aids (if any) are compounded with an unstabilized photosensitive resin, such as polypropylene, and the compounded resin compression molded into sheets approximately 20 mils in thickness. Several sheets of plastic are usually prepared in the above manner, each having different stabilizers, at different concentrations. Subsequent to formation of the foregoing compounded resin into sheet material, a series of dumb bells are cut from each sheet and placed in a Weather-Ohmeter. At 500 hours, or other pre-selected intervals, one (1) dumb bell of each sample of compounded resin is removed from the Weather-Ohmeter and its tensile strength measured on an Instron tensile testing device. The tensile strength of each sample is then compared to the values obtained from a sample cut from the same sheet which had not experienced any UV exposure. The sample is considered to have been photodegraded when it has experienced in excess of fifty percent (50%) reduction in its tensile strength.

## EXAMPLES

The Examples which follow further define, describe and illustrate the (i) process for the synthesis of polysubstituted α-amino-acetamide and (ii) evaluation of the stabilizer properties of the compounds obtained in such synthesis. Apparatus and procedures used in the foregoing process and evaluation of such samples are standard or as hereinbefore described. Parts and percentages appearing in such examples are by weight unless otherwise stipulated.

## EXAMPLE I

Preparation of α-(t-butylamino)isobutyramide - A three necked round bottomed flask, equipped with a reflux condensor, magnetic stirrer and a thermometer, is initially chilled by immersion of its base in an ice bath, and thereafer charged with 100 mmoles t-butylamine, 5 mmoles α-bromo-isobutyramide, 0.5 mmoles benzyltriethylammonium chloride. The flask is purged of air with an inert gas, such as argon, and 10 mmoles of 50% NaOH added dropwise to the charge over a period of ten minutes. After this initial period, the exotherm of the reaction plateaus. The contents of the flask are then heated and allowed to react overnight under refluxing conditions with mild agitation. Heating is thereafter discontinued, the contents of the flask diluted with water and the solids removed by filtration. The solids are thereafter washed with alternate solutions of water and methylene chloride for removal of reactant residues. Spectral and elemental analysis of the recovered product thus obtained are consistent with the structure of the title compound.

EXAMPLE II

The procedures of Examples I are repeated, except that the contents of the flask are heated under reflux conditions within one (1) hour after the addition of the NaOH to the charge, otherwise the synthesis proceeded essentially in the same manner. Upon completion of the reaction, the solids were separated from the charge by filtration. The filtrate was dried and concentrated to form an oil which yielded additional solids upon the addition of hexane. Spectral and elemental analysis of the product thus obtained are consistent with the structure of the title compound of Example I.

EXAMPLES III-XII

The procedures of Example I are repeated except for substituion of one or more, of the following reactants of Table I for those employed in Example I:

TABLE I

| EX. NO. | α-halo-acetamide | 1° or 2° amine | Yield(%) | mp(°C) |
|---|---|---|---|---|
| II | α-chloro-α,α-dimethyl-N-phenylacetamide | t-butylamine | 80 | 17.5-79 |
| III | α-bromo-α,α-dimethyl-N-phenylacetamide | t-butylamine | 82 | 77-79 |
| IV | α-bromo-α,α-dimethyl-N-t-butylacetamide | t-butylamine | 65 | 70-72 |
| V | α-bromo-α,α-dimethyl-acetamide | t-butylmine | 55 | 110.5-113 |
| VI | N,N'1,2-ethane-bis(α-chloro-α,α-dimethylacetamide) | t-butylamine | 80 | 96.5-98.5 |
| VII | α-bromo-α,α-dimethyl-N-2,2,6,6-tetramethyl-4-piperidinylacetamide | t-butylamine | 85 | 139-141 |
| VIII | α-bromo-α,α-dimethyl-N-2,2,6,6-tetramethyl-4-piperidinylacetamide | phenylamine | 84 | 163-164 |
| IX | α-bromo-α,α-dimethyl-N-t-butylacetamide | phenylamine | 71 | 140-142 |
| X | α-bromo-α,α-dimethyl-N-t-butylacetamide | morploline | 52 | 64-67 |

TABLE I (CONT)

| EX. NO | α-halo-acetamide | 1° or 2° amine | Yield (%) | mp(°C) |
|---|---|---|---|---|
| XI | α-bromo-α,α-dimethyl-N-2,2,6,6-tetramethyl-4-piperidinylacetamide | 4-amino-2,2,6,6-tetramethyl piperidine | 69 | 125-6 |
| XII | N',N-1,2-ethane-bis-(α-chloro-α,α-tetra-methylene-acetamide) | t-butylamine | 45 | 174-7 |

(*percentage based upon isolated product)

EXAMPLE XIII-XVIII

In order to ascertain the effectiveness as UV stabilizer of compounds prepared in accordance with the process of this invention, certain representative UV stabilizers are compounded with an unstabilized polypropylene resin, along with an antioxidant (GOODRITE 3125), compression molded into sheets approximately 20 mils thick and tested in a Weather-Ohmeter in the manner previously described. Table II, which follows, indicates the comparative effectiveness of the UV stabilizers subjected to such testing and further compares their performance to an unstabilized sample of the same resin.

TABLE II

| Ex. No. | Compound of ** | Failure of Sample * |
|---|---|---|
| XIII | Example VII | 1500 |
| XIV | Example VIII | 1200 |
| XV | Example VI | 1000 |
| XVI | Example XI | 1850 |
| XVII | Example V | 650 |
| XVIII | Control, (contains only antioxidant) | 180 |

*Sample considered to have failed upon loss of in excess of 50% of its tensile strength.

**All stabilizer compounds present in sample at concentration of 0.125 phr (parts per 100 parts resin); and antioxidant present at concentration of 0.10 phr (parts per 100 parts resin).

All of the stabilized films compare favorably, in terms of their resistance to photodegradation, to the unstabilized control.

The foregoing Examples have been provided as illustrative of a number of the preferred embodiments of this invention and are not intended for delineation of the scope thereof which is set forth in the following claims:

CLAIMS

1. In a process for the preparation of poly-substituted α-amino-acetamides of the formula

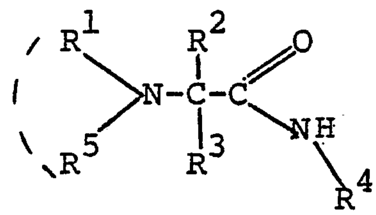

wherein $R^1$ and $R^4$ are independently selected from the group consisting of alkyl of 1-8 carbon atoms, aryl, cycloalkyl, piperidinyl, hindered piperidinyl and alkalene of 2-6 carbon atoms;

$R^2$ and $R^3$ are independently selected from the group consisting of hydrogen and alkyl of 1-6 carbon atoms; and

$R^5$ is hydrogen or alkyl of 1-6 carbon atoms and can, in conjunction with the acyclic substituents of $R^1$, form a heterocyclic group pendant from the α-carbon

by phase transfer catalyzed reaction, in a basic medium, of aniline, and its para-substituted derivatives, a primary or secondary amine and an oxirane compound;

the improvement comprising;

(a) combining, in an organic reaction medium and under an inert atmosphere, the following reagents:

(i) a stoichiometric excess of a primary or secondary amine of the formula

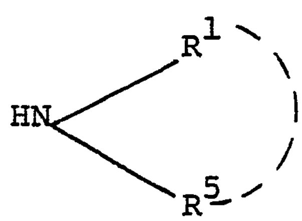

wherein $R^1$ is selected from the group consisting of alkyl of 1-8 carbon atoms, aryl, cycloalkyl, piperidinyl, partially or totally hindered piperidinyl, and alkalene of 2-6 carbon atoms, and

$R^5$ is hydrogen, alkyl of 1-6 carbon atoms and can, in conjunction with the acyclic substituents of $R^1$, form a heterocyclic compound.

(ii) an α-halo-acetamide of the formula

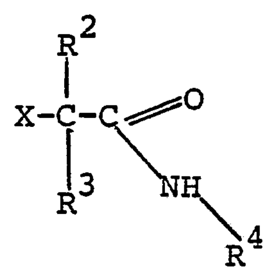

wherein $R^2$ and $R^3$ are independently selected from the group consisting of hydrogen and alkyl of 1-6 carbon atoms;

$R^4$ is selected from the group consisting of alkyl of 1-8 carbon atoms, aryl, cycloalkyl, piperidinyl, partially or totally hindered piperidinyl, and alkalene of 2-6 carbon atoms; and

X is chloride or bromide.

(iii) a stoichiometric excess of an aqueous solution of an alkali metal hydroxide; and

(iv) a catalytically effective amount of a phase transfer catalyst selected from among the onium salts of sulfur and the onium salts of the elements of Group VA of the periodic table; and

(b) allowing the reagents recited in step (a) to

react with one another under ambient laboratory conditions to form a polysubstituted α-amino-acetamide.

2. The process of claim 1, wherein the reaction temperature is in the range of from about 20°C to about 100°C.

3. The process of claim 1, wherein the molar ratio of primary or secondary amine to α-halo-acetamide is in the range of from about 1:1 to about 100:1.

4. The process of Claim 1, wherein the primary or secondary amine is selected from the group consisting of t-butylamine, t-octylamine, aniline, alkyl substituted aniline, isopropyl amine, propyl amine, 4-amino-2,2,6,6-tetramethyl-piperidine, allyl amine, morpholine, N-methyl-aniline, and diethyl amine.

5. The process of claim 1, wherein the α-halo-acetamide is selected from the group consisting of α-chloro--α,α-dimethyl-N-phenylacetamide, α-bromo-α,α-dimethyl-N-phenylacetamide, α-bromo-α,α-dimethyl-N-(t-butyl)acetamide, α-bromo-α,α-dimethylacetamide, N,N'[1,2-ethane-bis(α-bromo-α,α-diethyl acetamide)], and α-bromo-α,α-dimethyl-N-(2,2,6,6-tetramethyl-4-piperidinyl)acetamide.

6. The process of claim 1, wherein essentially only one product is formed by reaction of the reagents.

7. The process of claim 1 wherein the primary and secondary amine is selected from the group set forth in claim 4 and the α-haloacetamide is selected from the group set forth in claim 5.

8. A process which is directive for the synthesis of polysubstituted α-amino-acetamide from primary or secondary amines and α-halo-acetamides, said process comprising:

(a) combining, in an organic reaction medium and under an inert atmosphere, the following reagents:

(i) a stoichiometric excess of a primary or secondary amine of the formula

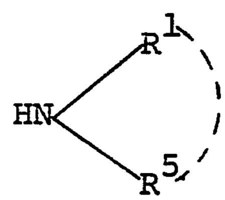

wherein $R^1$ is selected from the group consisting of alkyl of 1-8 carbon atoms, aryl, cycloalkyl, piperidinyl, partially or totally hindered piperidinyl, and alkalene of 2-6 carbon atoms, and $R^5$ is hydrogen, alkyl of 1-6 carbon atoms and can, in conjunction with the acyclic substituents of $R^1$, form a heterocyclic compound.

(ii) an α-halo-acetamide of the formula

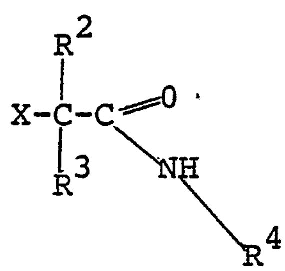

wherein $R^2$ and $R^3$ are independently selected from the group consisting of hydrogen and alkyl of 1-6 carbon atoms;

$R^4$ is selected from the group consisting of alkyl of 1-8 carbon atoms, aryl, cycloalkyl, piperidinyl, partially or totally hindered piperidinyl and alkalene of 2-6 carbon atoms, and

X is chloride or bromide.

(iii) a stoichiometric excess of an aqueous solution of an alkali metal hydroxide; and

(iv) a catalytically effective amount of a phase transfer catalyst selected from among the onium salts of sulfur and the onium salts of the elements of Group VA of the periodic table; and

(b) allowing the reagents recited in step (a) to react with one another under ambient laboratory conditions, whereby a single polysubstituted α-amino-acetamide is formed.

9. The process of claim 8, wherein the α-halo-acetamide is converted to an intermediate compound of the formula

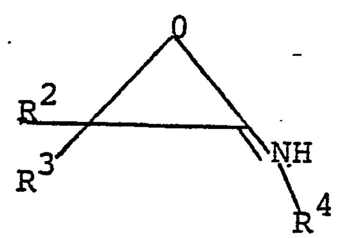

10. The process of claim 9, wherein the intermediate compound reacts with the primary or secondary amine to form the polysubstituted α-amino-acetamide.

11. The process of claim 8, wherein the primary or secondary amine is selected from the group consisting of t-butylamine, t-octylamine, aniline, alkyl substituted aniline, isopropyl amine, propyl amine, 4-amino-2,2,6,6-tetramethyl-piperidine, allyl amine; and, morpholine, N-methyl-aniline, diethyl amine.

12. The process of claim 8, wherein the α-halo-acetamide is selected from the group consisting of α-chloro-α,α-dimethyl-N-phenylacetamide, α-bromo-α,α-dimethyl-N-phenylacetamide, α-bromo-α,α-dimethyl-N-(t-butyl)acetamide, α-bromo-α,α-dimethylacetamide, N,N'[1,2-ethane-bis(α-bromo-α,α-diethyl acetamide)], and α-bromo-α,α-dimethyl-N-(2,2,6,6-

tetramethyl-4-piperidinyl)acetamide.

13. The process of claim 8, wherein the reaction temperature is in the range of from about 20°C to about 100°C.

14. The process of claim 8, wherein the molar ratio of primary or secondary amine to α-halo-acetamide is in the range of from about 10:1 to about 100:1.

15. The process of claim 8, wherein the primary and secondary amine is selected from the group of claim 11, and the α-ammoacetamide is selected from the group of claim 12.

European Patent Office

# EUROPEAN SEARCH REPORT

0062322

Application number

EP 82 10 2819

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | EP-A-0 007 182 (GOODRICH) *Claims* --- | 1 | C 07 C 102/00<br>C 07 C 103/50<br>C 07 D 211/58<br>C 07 D 295/18 |
| Y | FR-A-2 118 985 (ASTRA PHARMACEUTICAL PRODUCTS) *Claim 4* & US - A - 3 812 147 --- | 1 | |
| Y | CHEMICAL ABSTRACTS, vol. 95, no. 1, 6th July 1981, page 684, no. 7214t, Columbus Ohio (USA); T.OKAWARA et al.: "Convenient syntheses of piperazine-2,5-diones and lactams from halocarboxamides using phase transfer catalysts". & CHEM. LETT. 1981, (2), 185-8. *Abstract* --- | 1 | TECHNICAL FIELDS SEARCHED (Int. Cl. 3) |
| Y | CHEMICAL ABSTRACTS, vol. 91, no. 5, 30th July 1979, page 614, no. 39275x, Columbus Ohio (USA); K.M.PATEL et al.: "Phase transfer catalysis: reaction of 4-bromopyridine with cyclic secondary amines". & SYNTH. COMMUN. 1979, 9(4), 251-3. *Abstract* ----- | 1 | C 07 C 102/00<br>C 07 C 103/00<br>C 07 D 211/00<br>C 07 D 295/00 |

The present search report has been drawn up for all claims

| Place of search | Date of completion of the search | Examiner |
|---|---|---|
| THE HAGUE | 23-06-1982 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document